# EUROPEAN PATENT APPLICATION

(11) **EP 4 406 539 A1**
(43) Date of publication of application: **31.07.2024**
(21) Application number: 23382055.4
(22) Date of filing: 24.01.2023
(51) Int. Cl.: A61K 31/567, A61K 9/20, A61P 15/00, A61P 23/00, A61P 29/00

(54) **LOW-DOSE MIFEPRISTONE FOR THE TREATMENT OF ENDOMETRIOSIS ASSOCIATED PAIN**

(71) Applicant: Laboratorios Litaphar, S.L., 20730 Azpeitia - Gipuzkoa (ES)
(72) Inventor: TOMASI SCIANÒ, Giuseppe Giovanni, E-20730 Azpeitia, Gipuzkoa (ES); LARRAÑAGA LAZCANO, Lidia, E-20730 Azpeitia, Gipuzkoa (ES); LARRAÑAGA LAZCANO, Ander, E-20730 Azpeitia, Gipuzkoa (ES); RUIZ OSANTE, Borja, E-48012 Bilbao, Vizcaya (ES)
(74) Representative: ABG Intellectual Property Law, S.L.

(57) **Abstract**

The present invention relates to the use of the compound mifepristone or a pharmaceutically acceptable salt, solvate or active metabolite thereof at a low dose in the treatment and/or prevention of endometriosis associated pain.

## Description

### Field of the Invention

The present invention relates to the management of endometriosis associated pain, more particularly, to the use of mifepristone and pharmaceutical compositions thereof against this condition.

### Background of the Invention

Endometriosis is a highly complex, livelong disease of unknown cause affecting approximately 10% (190 million) of reproductive age women that is characterized by the presence of tissue resembling endometrium (the lining of the uterus) outside the uterus (e.g., the ovaries and other pelvic structures such as the fallopian tubes, peritoneum, vagina or intestines).

It causes a chronic inflammatory reaction that may result in the formation of scar tissue (adhesions, fibrosis) within the pelvis and other parts of the body. Several lesion types have been described including superficial endometriosis found mainly on the pelvic peritoneum; cystic ovarian endometriosis (endometrioma) found in the ovaries; deep endometriosis found in the recto-vaginal septum, bladder, and bowel; in some cases, endometriosis has also been found outside the pelvis.

Symptoms associated with endometriosis vary and include: painful periods (dysmenorrhea), chronic pelvic pain, painful sexual intercourse (dyspareunia), painful bowel movements, painful urination (dysuria), infertility, fatigue, depression or anxiety, abdominal bloating and nausea. The severity of endometriosis symptoms, however, is not correlated with the severity of endometriosis or the stage of endometriosis. Indeed, some women with extensive endometriosis are asymptomatic, while some women with minimal disease have incapacitating pain. Further, in certain patients, the only symptom may be infertility with no painful episodes. However, if present, the pain associated with endometriosis is the most difficult symptom to cope with for most women because its detrimental consequences on the quality of life.

Despite the prevalence of endometriosis and its impact on public health, there is currently no known way to prevent or cure it.

Treatment can be with medications and/or surgery depending on symptoms, lesions, desired outcome, and patient choice. In most women a combination of surgical and medical treatment is required, often with side effects and variable efficacy.

Surgery can remove endometriosis lesions, adhesions, and scar tissue. However, the success of surgical removal depends on the extent of disease and on the skill of the surgeon. In addition, lesions may recur even after successful eradication, and pelvic floor muscle abnormalities can contribute to chronic pelvic pain. Besides, excision of endometriomas adversely affects ovarian follicular reserve. Thus, for women who want to preserve their fertility, the potential benefits of surgery should be weighed against these negative effects. Further, in the most severe cases, conservative surgery (e.g. laparoscopy) is not useful but the only surgical option is doing a hysterectomy with removal of the uterus and both ovaries, which involves loss of fertility apart from the associated risk of surgical morbidity and high cost.

Current medical therapies for endometriosis are essentially based on two mechanisms of action: anti-inflammatory and hormonal.

When endometriosis-induced pain is present, non-steroidal anti-inflammatory drugs (NSAIDs) such as ibuprofen or naproxen may be used for temporary relief of this symptom. NSAIDs work on endometriosis pain by blocking the development of prostaglandins. Inevitable dose increases (for episodes of acute incapacitating pain) or extended dosing periods (for management of chronic pain) may lead to undesirable outcomes, including gastro-intestinal and cardiovascular side effects.

While opioids can also be used for managing pain, it has been reported that women with endometriosis that are prescribed opioids for pain relief are at a high risk for chronic opioid use, dependence and overdose.

By contrast, the hormonal treatment of endometriosis is not directly focused on analgesia but aims at suppressing ovarian function and endometrial tissue growth. Hormonal drugs used or studied for the treatment of endometriosis are combined hormonal contraceptives, progestogens and anti-progestogens (also known as progestins and anti-progestins, respectively), gonadotropin releasing hormone (GnRH) agonists and antagonists, and aromatase inhibitors.

Combined hormonal contraceptives (CHCs), containing two types of hormones, an estrogen and a progestogen, are available as pills (combined oral contraceptive pill), skin patches and vaginal rings. CHCs help control the hormones responsible for the buildup of endometrial tissue each month.

Progestogens (e.g., medroxyprogesterone acetate, oral or depot, dienogest, cyproterone acetate, norethisterone acetate, danazol, levonorgestrel intrauterine device) and anti-progestogens (e.g., gestrinone) can halt menstrual periods and the growth of endometrial implants, which may relieve endometriosis signs and symptoms.

Gonadotropin-releasing hormone (Gn-RH) agonists (e.g., leuprolide acetate, goserelin acetate, and nafarelin) and antagonists (e.g. elagolix) block the production of ovarian-stimulating hormones, lowering estrogen levels and preventing menstruation, thus creating an artificial menopause.

Aromatase inhibitors (e.g., anastrazole, fadrozole, formestane, exemestane, letrozole) are a class of medicines that also reduce the amount of estrogen in the body. Localized aromatase production and resulting estradiol formation by endometriotic lesions have prompted the successful off-label use of aromatase inhibitors for women with symptoms that are resistant to hormone therapy.

However, the prolonged hypoestrogenic state which develops during treatment with most of the above hormonal agents may also result in undesirable outcomes, including decrease in bone density, development of osteoporosis, and menopausal symptoms such as amenorrhea, hot flashes, mood swings, etc. For this reason, long-term use is not recommended. For instance, treatment with GnRH agonists is limited by approved labeling to 6 months and danazol therapy may last up to 9 months.

Selective progesterone receptor modulators (SPRMs) are synthetic steroids that have both antagonist and agonist properties when binding to progesterone receptors. They induce amenorrhea with no hypoestrogenic side effects and allow for a reduction in uterine bleeding. Mifepristone (CAS number 84371-65-3) is a practically pure SPRM approved for the termination of pregnancy, cervical dilation, medical termination of pregnancy during the second trimester, and fetal death in utero.

Several clinical studies have investigated mifepristone in the treatment of endometriosis. In one of the trials, six normally cycling women with endometriosis were given orally 100 mg mifepristone daily for three months. Amenorrhea was reported by all study participants; all women also reported improvement in pelvic pain. However, no significant change in the extent of endometriosis was evident from follow-up laparoscopy (Kettel L M, et al. Endocrine responses to long-term administration of the anti progesterone RU486 in patients with pelvic endometriosis. Fertil Steril 1991; 56:402-7).

In another clinical trial, nine women with endometriosis received mifepristone orally, 50 mg daily for 6 months. Pelvic pain and uterine cramping improved in all patients and endometriosis regressed by 55 % as evidenced by laparoscopic assessments of endometrial implants using the American Fertility Society (AFS) score. All patients exhibited amenorrhea without hypoestrogenism. Elevation of liver transaminases was observed in one subject (Kettel L M, et al. Treatment of endometriosis with the anti progesterone mifepristone (RU486). Fertil Steril 1996; 65:23-8).

When the same group of researchers conducted a third clinical study in seven women with endometriosis using oral dose of mifepristone of 5 mg daily administered for 6 months, pelvic pain improved in six of seven patients and cyclic bleeding ceased in all patients; however, four women complained of irregular bleeding and mean endometriosis scores decreased by only 20 %. Based on these data, the authors recommended the 50 mg mifepristone dose (Kettel L M, et al. Preliminary report on the treatment of endometriosis with low-dose mifepristone (RU 486). Am J Obstet Gynecol 1998; 178:1151-6). Nevertheless, such a high dose may lead to the increased risk of endometrial hyperplasia and clinically significant elevations in liver enzymes.

A more recent clinical trial (ClinicalTrials.gov Identifier NCT02271958) completed in 2013 evaluated the effectiveness and safety of 2.5, 5 and 10 mg doses of oral mifepristone against a placebo in 360 women with laparoscopic diagnostic of endometriosis. All mifepristone treatment groups were more effective than the placebo group. However, while after 90 and 180 days of treatment no differences were observed between 5 and 10 mg doses which behave similarly with regard to clinical improvement, there was a statistically significant difference in favor of groups 2 and 3 (5 and 10 mg) with respect to group 1 (2.5 mg), regarding the improvement of all symptoms. This difference concurs with the significantly greater use of analgesics sustained by the subjects in group 1 in comparison with the groups 2 and 3. The fact that 2.5 mg group presented significantly lower percentages of amenorrhea and clinical improvement than the 5 and 10 mg groups, along with the necessity of administering higher amounts of analgesics as rescue therapy in the 2.5 mg patients, led the authors to propose the 5 mg dose as efficacious and safe option for the treatment of endometriosis which should be the studied in future trials (Carbonell J L et al. Mifepristone 2.5, 5, 10 mg versus placebo in the treatment of endometriosis. Journal of Reproductive Health and Medicine 2016; 2(1): 17-25).

Therefore, the above studies on the treatment of endometriosis with mifepristone show that treating the disease (e.g., reducing the size and the number of endometrial lesions) does not necessarily treat pain associated with endometriosis or *vice versa.* Indeed, as previously noted, it is widely known that there is a lack of correlation between the disease severity/stage and the symptoms associated with the same. Specifically, pain, which is the central symptom in endometriosis, often persists despite adequate treatment of the disease, even after surgical removal of the endometrial lesions.

Thus, there is still a great need of providing new therapies suitable for the treatment and/or prevention of pain associated with endometriosis.

### Summary of the Invention

Described herein is a novel treatment for endometriosis associated pain. In particular, we demonstrate that the compound mifepristone at low-doses is able to reduce satisfactorily the pain associated with endometrial lesions without the aid of further analgesics (rescue therapy). Beneficial pain relief has been observed even when endometriosis is found in extra-pelvic sites.

Unexpectedly, the use of a daily dosage of 2.5 mg mifepristone as single medication (monotherapy) provided better relief of endometriosis associated pain in comparison with higher dosages. The use of such an extremely low dose of mifepristone, which additionally may be administered alone, allows an effective treatment of endometriosis associated pain with less potential side effects compared to regimens proposed in prior art studies for treating endometriosis, which either propose higher doses of mifepristone or require the co-administration of analgesics. Needless to say that this unpredictable finding results in important cost savings for patients and health services.

Therefore, the present invention relates to mifepristone or a pharmaceutically acceptable salt, solvate or active metabolite thereof for use in the treatment and/or prevention of endometriosis associated pain, wherein said mifepristone or a pharmaceutically acceptable salt, solvate or active metabolite thereof is used as the sole analgesic agent at a daily dose of between 2 mg and 3 mg.

A further aspect is a pharmaceutical composition for use in the treatment and/or prevention of endometriosis associated pain, said composition comprising mifepristone, or a pharmaceutically acceptable salt, solvate or active metabolite thereof and a pharmaceutically acceptable excipient, and wherein said mifepristone or a pharmaceutically acceptable salt, solvate or active metabolite thereof is used as the sole analgesic agent at a daily dose of between 2 mg and 3 mg.

A further aspect is a pharmaceutical composition in the form of a tablet for oral administration comprising:
Mifepristone or a pharmaceutically acceptable salt, solvate or active metabolite thereof 2 - 3 mg
Microcrystalline cellulose 77 - 95 mg
Polyvinylpyrrolidone 2 - 6 mg
Croscarmellose sodium 1 - 5 mg
Anhydrous colloidal silica 0 - 1 mg
Magnesium stearate 0 - 2 mg.

A further aspect is a pharmaceutical composition in the form of a tablet for oral administration comprising:
Mifepristone or a pharmaceutically acceptable salt, solvate or active metabolite thereof, between more than 2 % w/w and less than 3 % w/w
Microcrystalline cellulose 76 - 94 % w/w
Polyvinylpyrrolidone 2 - 6 % w/w
Croscarmellose sodium 1 - 5 % w/w
Anhydrous colloidal silica 0 -1 % w/w
Magnesium stearate 0 - 2 % w/w.

Another aspect of the present invention relates to a kit comprising mifepristone or a pharmaceutically acceptable salt, solvate or active metabolite thereof, preferably provided as a pharmaceutical composition, and instructions for use in the treatment and/or prevention of endometriosis associated pain at a daily dose of between 2 mg and 3 mg of mifepristone or a pharmaceutically acceptable salt, solvate or active metabolite thereof.

Another aspect of the present invention relates to a method for the treatment and/or prevention of endometriosis associated pain in a subject, said method comprising the administration of mifepristone, or a pharmaceutically acceptable salt, solvate or active metabolite thereof at a daily dose of between 2 mg and 3 mg as the sole analgesic agent to said subject.

Another aspect of the present invention relates to the use of mifepristone or a pharmaceutically acceptable salt, solvate or active metabolite thereof for the manufacture of a medicament for the treatment and/or prevention of endometriosis associated pain, wherein said mifepristone or a pharmaceutically acceptable salt, solvate or active metabolite thereof is used as the sole analgesic agent at a daily dose of between 2 mg and 3 mg.

Another aspect of the present invention relates to a dose regimen for providing analgesia against endometriosis associated pain in a subject, the regimen comprising administering mifepristone, or a pharmaceutically acceptable salt, solvate or active metabolite thereof as the sole analgesic agent at a daily dose of between 2 mg and 3 mg.

According to a particular embodiment, the compounds, compositions, kits, methods, uses and dose regimens described herein relate to oral administration. In another embodiment, the subject to be treated is a woman.

These aspects and preferred embodiments thereof are additionally also defined hereinafter in the detailed description and in the claims.

### Brief Description of the Drawings

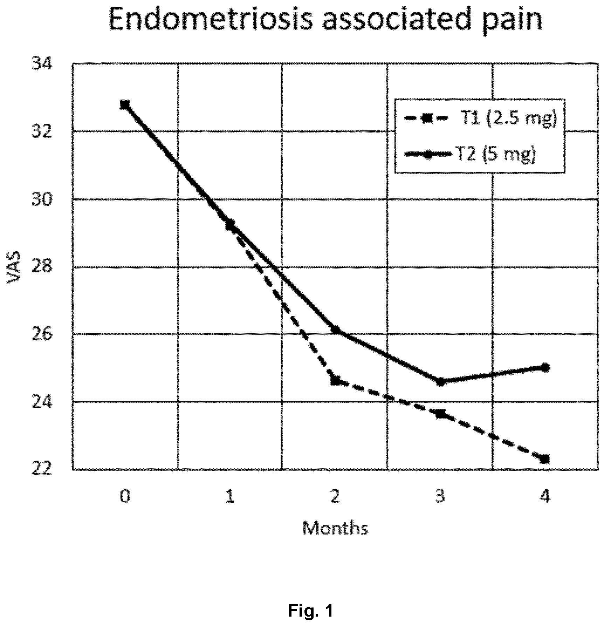

***Figure 1*****.** Graph showing endometriosis associated pelvic pain experienced by two group patients using the visual analogue scale (VAS) vs time in months. Group T1 corresponds to a treatment with 2.5 mg mifepristone per day whereas Group T2 corresponds to a treatment with 5 mg mifepristone per day.

### Detailed Description of the invention

Endometriosis is a clinically important and highly prevalent, but only poorly understood disease. It occurs when cells from the mucus membrane lining the uterus (endometrium) form implants that attach, grow, and function outside the uterus, generally in the pelvic region but also in extra-pelvic locations.

The endometriosis-associated pain is the most difficult symptom to cope with for most women. For many women, the pain they suffer severely interferes with everyday life.

In this regard, non-steroidal anti-inflammatory drugs (NSAIDs) such as ibuprofen or naproxen are most commonly used as first-line treatment for women with pain associated with endometriosis (also paracetamol). However, the administration of NSAIDs at escalating doses for combating episodes of acute incapacitating pain and/or during extended periods for the management of chronic pain come with several risks including gastrointestinal bleeding, increased risk of heart attack or stroke, skin issues such as bruising, bleeding, or rash, allergic reactions, etc.

Given the limited effectiveness of available treatments, a significant number of patients with endometriosis pain resort to use of opioid to manage their pain. However, the addictive and euphoric properties of opioids put patients at risk of abuse, misuse, addiction and even death. And long-term use for chronic pain can lead to opioid tolerance, where diminishing pain relief is observed by patients, who then require regular increases in doses to maintain meaningful pain relief. Thus, prescribing opioids for the treatment of chronic (long-term) pain, like in the case of endometriosis, has become discouraged.

Several clinical studies have investigated mifepristone in the treatment of endometriosis. However, these studies recommended the use of high doses of mifepristone (> 50 mg) that may lead to dangerous side effects or, when lower doses are administered, it turned out to be necessary to intake analgesic rescue medication such as ibuprofen or other NSAIDs in order to properly reduce the pain associated with endometriosis.

The compounds, compositions, kits, methods, uses and dose regimens described herein find use in the treatment, prevention or both of endometriosis associated pain.

As used herein, endometriosis associated pain refers to any type of pain associated with endometriosis including, but not being limited to, nonmenstrual pelvic and extra-pelvic pain, dysmenorrhea, dyschezia and dyspareunia.

In a particular embodiment, the endometriosis associated pain is associated with extra-pelvic endometriosis such as pain experienced by patients with endometrial lesions in cesarean sections (C-sections) or ovarian endometriomas.

As used herein, the terms "treat", "treating" and "treatment" include in general the eradication, removal, reversion, alleviation, modification, or control of endometriosis associated pain.

As used herein, the terms "prevention", "preventing", "preventive", "prevent" and "prophylaxis" refer to the capacity of a given substance, composition or medicament to avoid, minimize or difficult the onset or development of endometriosis associated pain before its onset.

This invention provides for the long term use of mifepristone at a low dose, namely between 2 mg and 3 mg, to treat and/or prevent endometriosis associated pain. The invention is advantageous over the prior art because the prolonged use of the recited low dose of mifepristone as a single therapy results in improvements in the patient's condition with a lower risk of experiencing clinically adverse side effects. At the same time, treatment costs go down.

In one aspect, the invention refers to mifepristone at a therapeutically effective amount of between 2 mg and 3 mg per day for use in treating and/or preventing endometriosis associated pain, with the proviso that mifepristone is the one and only analgesic agent used.

The present invention also contemplates all pharmaceutically acceptable salts, solvates and active metabolites of mifepristone.

By "pharmaceutically acceptable" is meant herein a material that is not biologically or otherwise undesirable, i. e., the material may be incorporated into a pharmaceutical composition administered to a patient without causing any undesirable biological effects or interacting in a deleterious manner with any of the other components of the composition in which it is contained. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

The term "salt" must be understood as any form of mifepristone used according to this invention in which said compound is in ionic form or is charged and coupled to a counterion (a cation or anion).

The term "solvate" is to be understood as meaning any form of the mifepristone used according to this invention in which this compound has attached to it via non-covalent binding another molecule (most likely a polar solvent) especially including hydrates and alcoholates, e.g. methanolate. Methods of solvation are generally known within the art. For instance, CN106317152A discloses a n-propanol solvate of mifepristone and Xu et al (Xu, Juan, Li, Peng, Wang, Hui P. and Ning, Li F.. "Crystal structure of 11-(4-(dimethylamino)phenyl)-17-hydroxy-13-methyl-17-(prop-1-yn-1-yl)-1,2,6,7,8,11,12,13,14,15,16,17-dodecahydro-3H-cyclopenta[a]phenanthren-3-one - acetonitril (1/2), C33H41N3O2" Zeitschrift für Kristallographie - New Crystal Structures, vol. 232, no. 4, 2017, pp. 573-575) discloses the acetonitrile solvate, both being contemplated in the present invention.

An "active metabolite", as used herein, refers to a product produced through metabolism in the body of a specified compound or salt or solvate thereof and which exhibits the same biological activity as the specified compound. Active metabolites of mifepristone or of a salt or solvate thereof may be identified using routine techniques known in the art and their activities determined using tests such as those described herein. Such metabolites may result for example from the oxidation, reduction, hydrolysis, amidation, deamidation, esterification, deesterification, enzymatic cleavage, and the like, of the administered mifepristone or of a salt or solvate thereof. Accordingly, the invention includes active metabolites of mifepristone or of a salt or solvate thereof, including compounds produced by a process comprising contacting mifepristone or of a salt or solvate thereof with a mammal for a period of time sufficient to yield a metabolic product thereof. Such metabolite may also be produced in vitro by oxidation, reduction, hydrolysis, amidation, deamidation, esterification, deesterification, or enzymatic cleavage of the corresponding mifepristone or salt or solvate thereof.

Examples of metabolites of mifepristone (RU 486) include those described in Shi et al (Shi YE, Ye ZH, He CH, Zhang GQ, Xu JQ, Van Look PF, Fotherby K. Pharmacokinetic study of RU 486 and its metabolites after oral administration of single doses to pregnant and non-pregnant women. Contraception 1993; 48(2): 133-49), e.g. RU 42633 (monodemethyl metabolite), RU 42848 (didemethyl metabolite) and RU 42698 (hydroxyl metabolite). These are the 3 most proximal metabolites of mifepristone and all retain considerable affinity toward the human progesterone and glucocorticoid receptors; in addition, the serum concentrations of these 3 metabolites are in a similar range as those of the parent drug.

The invention also refers to pharmaceutical compositions or medicaments, based on mifepristone 2 mg and 3 mg per day, for use in treating and/or preventing endometriosis associated pain, with the proviso that mifepristone is the one and only analgesic agent used.

The compositions can for example be administered orally, topically, dermally, nasally, intravenously, intramuscularly, intraperitoneally, intracerobrospinally, intracranially, intraspinally, subcutaneously, intraarticularly, intrasynovialy, or intrathecaly. Other forms of administration are not excluded. According to a particular embodiment, said composition is for oral administration.

Examples of pharmaceutical compositions include any solid composition (e.g. tablets, pills, capsules, granules) or liquid composition (e.g. solutions, suspensions lotions or emulsions).

In a particular embodiment, said composition is formulated as a tablet or capsule for oral administration. Capsules are solid dosage forms using preferentially either a hard or soft gelatin shell as a container for the mixture of mifepristone and excipients. Oral pharmaceutical compositions are preferentially compressed tablets.

The respective composition may - depending on its route of administration - also contain one or more excipients known to those skilled in the art. The composition or medicament according to the present invention may be produced according to standard procedures known to those skilled in the art.

The term "excipient" refers to components of a drug compound other than the active ingredient (definition obtained from the European Medicines Agency-EMA). They preferably include a "carrier, adjuvant and/or vehicle". Carriers are forms to which substances are incorporated to improve the delivery and the effectiveness of drugs. Drug carriers are used in drug-delivery systems such as the controlled-release technology to prolong in vivo drug actions, decrease drug metabolism, and reduce drug toxicity. Carriers are also used in designs to increase the effectiveness of drug delivery to the target sites of pharmacological actions (U.S. National Library of Medicine. National Institutes of Health). Adjuvant is a substance added to a drug product formulation that affects the action of the active ingredient in a predictable way. Vehicle is an excipient or a substance, preferably without therapeutic action, used as a medium to give bulk for the administration of medicines (Stedman's Medical Spell checker, © 2006 Lippincott Williams & Wilkins). Such pharmaceutical carriers, adjuvants or vehicles can be sterile liquids, such as water and oils, including those of petroleum, animal, vegetable or synthetic origin, such as peanut oil, soybean oil, mineral oil, sesame oil and the like, excipients, disgregants, wetting agents or diluents. Suitable pharmaceutical carriers are described in "Remington's Pharmaceutical Sciences" by E.W. Martin. The selection of these excipients and the amounts to be used will depend on the form of application of the pharmaceutical composition.

In an embodiment, the pharmaceutical composition comprises one or more diluents, such as microcrystalline cellulose. In an embodiment, the pharmaceutical composition comprises one or more binders, such as polyvinylpyrrolidone. In an embodiment, the pharmaceutical composition comprises one or more disintegrants, such as croscarmellose sodium. In an embodiment, the pharmaceutical composition comprises one or more glidants, such as anhydrous colloidal silica. In an embodiment, the pharmaceutical composition comprises one or more lubricants, such as magnesium stearate.

In a more particular embodiment, the pharmaceutical composition, e.g. a tablet for oral use, comprises mifepristone or a pharmaceutically acceptable salt, solvate or active metabolite thereof in an amount between 2 mg and 3 mg in combination with at least one pharmaceutically acceptable excipient including, but not being limited to, suitable diluents (e.g. microcrystalline cellulose) and/or binders (e.g polyvinylpyrrolidone) and/or disintegrants (e.g. croscarmellose sodium) and/or glidants (e.g. anhydrous colloidal silica) and/or lubricants (e.g. magnesium stearate).

More particularly, a pharmaceutical composition, preferably in the form of an oral tablet, according to the present invention may comprise mifepristone or a pharmaceutically acceptable salt, solvate or active metabolite thereof in an amount between 2 mg and 3 mg (e.g. about 2.5 mg) and at least one of:
a diluent, such as microcrystalline cellulose, 77 - 95 mg (e.g. about 90.95 mg)
a binder, such as polyvinylpyrrolidone, 2 - 6 mg (e.g. about 4.00 mg)
a disintegrant, such as croscarmellose sodium, 1 - 5 mg (e.g. about 3.20 mg)
a glidant, such as anhydrous colloidal silica, 0 - 1 mg (e.g. about 0.30 mg)
a lubricant, such as magnesium stearate, 0 - 2 mg (e.g. about 1.05 mg).

In a preferred embodiment, an oral tablet according to the present invention comprises or consists of:
Mifepristone or a pharmaceutically acceptable salt, solvate or active metabolite thereof 2 - 3 mg
Diluents 77 - 95 mg
Binders 2 - 6 mg
Disintegrants 1 - 5 mg
Glidants 0 - 1 mg
Lubricants 0 - 2 mg.

In a more preferred embodiment, an oral tablet according to the present invention comprises or consists of:
Mifepristone or a pharmaceutically acceptable salt, solvate or active metabolite thereof 2 - 3 mg (e.g. about 2.5 mg)
Microcrystalline cellulose 77 - 95 mg (e.g. about 90.95 mg) Polyvinylpyrrolidone 2 - 6 mg (e.g. about 4.00 mg)
Croscarmellose sodium 1 - 5 mg (e.g. about 3.20 mg)
Anhydrous colloidal silica 0 - 1 mg (e.g. about 0.30 mg)
Magnesium stearate 0 - 2 mg (e.g. about 1.05 mg).

In another particular embodiment, the pharmaceutical composition, e.g. a tablet for oral use, comprises mifepristone or a pharmaceutically acceptable salt, solvate or active metabolite thereof in an amount between more than 2 % w/w and less than 3 % w/w in combination with at least one pharmaceutically acceptable excipient including, but not being limited to, suitable diluents (e.g. microcrystalline cellulose) and/or binders (e.g polyvinylpyrrolidone) and/or disintegrants (e.g. croscarmellose sodium) and/or glidants (e.g. anhydrous colloidal silica) and/or lubricants (e.g. magnesium stearate).

More particularly, a pharmaceutical composition, preferably in the form of an oral tablet, according to the present invention may comprise mifepristone or a pharmaceutically acceptable salt, solvate or active metabolite thereof in an amount between more than 2 % w/w and less than 3 % w/w (e.g. about 2.45 % w/w) and at least one of:
a diluent, such as microcrystalline cellulose, 76 - 94 % w/w (e.g. about 89.17%)
a binder, such as polyvinylpyrrolidone, 2 - 6 % w/w (e.g. about 3.92%)
a disintegrant, such as croscarmellose sodium, 1 - 5 % w/w (e.g. about 3.14%)
a glidant, such as anhydrous colloidal silica, 0 -1 % w/w (e.g. about 0.29%)
a lubricant, such as magnesium stearate, 0 - 2 % w/w (e.g. about 1.03%).

In a preferred embodiment, an oral tablet according to the present invention comprises or consists of:
Mifepristone or a pharmaceutically acceptable salt, solvate or active metabolite thereof, above 2 % w/w and below 3 % w/w
Diluents 76 - 94 % w/w
Binders 2 - 6 % w/w
Disintegrants 1 - 5 % w/w
Glidants 0 -1 % w/w
Lubricants 0 - 2 % w/w.

In a more preferred embodiment, an oral tablet according to the present invention comprises or consists of:
Mifepristone or a pharmaceutically acceptable salt, solvate or active metabolite thereof, above 2 % w/w and below 3 % w/w (e.g. about 2.45%)
Microcrystalline cellulose 76 - 94 % w/w (e.g. about 89.17%) Polyvinylpyrrolidone 2 - 6 % w/w (e.g. about 3.92%)
Croscarmellose sodium 1 - 5 % w/w (e.g. about 3.14%)
Anhydrous colloidal silica 0 -1 % w/w (e.g. about 0.29%)
Magnesium stearate 0 - 2 % w/w (e.g. about 1.03%).

The pharmaceutical compositions of the invention are primarily intended for use in the treatment and/or prevention of endometriosis associated pain, being preferably administered to provide a daily dose of between 2 mg and 3 mg (e.g. about 2.5 mg) of mifepristone or a pharmaceutically acceptable salt, solvate or active metabolite thereof. Nevertheless, while particularly useful for managing endometriosis associated pain, the pharmaceutical compositions described herein are also suitable against other diseases known to be treatable and/or preventable by mifepristone.

In another aspect, the present invention refers to a method for preparing the pharmaceutical composition of the invention. For instance, the tablets for oral administration described herein may be prepared by a direct compression step of the components.

In an embodiment, tablets are produced by the formation of a premix wherein the active ingredient is mixed with an equal amount of diluent (e.g. microcrystalline cellulose) and is sieved. In the meantime, the remaining diluent, binder (e.g polyvinylpyrrolidone) and disintegrant (e.g. croscarmellose sodium) are sieved and mixed together. To this mixture is added and mixed the premix containing the active ingredient. Lastly, the lubricants (e.g. magnesium stearate) and glidants (e.g. anhydrous colloidal silica) are added and this mixture is compressed. The mixture is compressed to obtain tablets, which may for instance have an average weight of about 102 mg with active ingredient content equal to about 2.5 mg.

In a more particular embodiment, the process for preparing tablets comprises the following steps:
a) forming a premix by mixing mifepristone or a pharmaceutically acceptable salt, solvate or active metabolite thereof with an equal amount of microcrystalline cellulose and sieving;
b) sieving and mixing together the remaining microcrystalline cellulose, polyvinylpyrrolidone and croscarmellose sodium to form a mixture;
c) combining the premix of step (a) and the mixture of step (b) to form a mixture;
d) adding magnesium stearate and anhydrous colloidal silica to the mixture of step c) to form a further mixture; and
e) compressing the aforementioned mixture of step d) into tablets.

Any or all of the above described embodiments of the pharmaceutical composition may be combined with each other to arrive at new embodiments. The expression % w/w refers to weight percentage relative to the total weight of the composition considered. It is to be understood that the weight percentages of each component are chosen such that the total percentage does not exceed 100% with respect to the total weight of the pharmaceutical composition. In an embodiment, the weight percentages of each component are chosen such that the total percentage does not reach 100% with respect to the total weight of the pharmaceutical composition, such that further components may be present. In an embodiment, the weight percentages of each component are chosen such that the total percentage totals 100% with respect to the total weight of the pharmaceutical composition, such that further components are not present, in which case the pharmaceutical composition consists of such components.

The present invention also refers to a kit for use in treating and/or preventing endometriosis associated pain comprising:
a) the compound mifepristone or a pharmaceutically acceptable salt, solvate or active metabolite thereof, preferably provided as a pharmaceutical composition as described herein, and
b) instructions for use, for example written instructions on how to administer the compound or the composition at a daily dose of between 2 mg and 3 mg;

in a suitable container and/or with suitable packaging.

Also is disclosed a method of treatment of a patient suffering endometriosis associated pain, or likely to suffer endometriosis associated pain, which comprises administering to the patient in need of such a treatment or prophylaxis a therapeutically effective amount of between 2 mg and 3 mg of mifepristone, or a pharmaceutically acceptable salt, solvate or active metabolite thereof per day as the one and only analgesic agent.

According to the present invention, the patient or subject is a female mammal, notably, a woman.

Reference herein to a "subject likely to suffer endometriosis associated pain" is intended to refer to a subject at higher risk of developing pain associated with endometriosis compared to the general population.

By an "effective" amount or a "therapeutically effective amount" of a drug or pharmacologically active agent is meant a nontoxic but sufficient amount of the drug or agent to provide the desired effect. In the therapy of the present invention, an "effective amount" of mifepristone or a pharmaceutically acceptable salt, solvate or active metabolite thereof is the amount of that compound that is effective to provide relief of endometriosis associated pain, namely between 2 mg and 3 mg daily, e.g. about 2.0 mg, about 2.05 mg, about 2.1 mg, about 2.15 mg, about 2.2 mg, about 2.25 mg, about 2.3 mg, about 2.35 mg, about 2.4 mg, about 2.45 mg, about 2.5 mg, about 2.55 mg, about 2.6 mg, about 2.65 mg, about 2.7 mg, about 2.75 mg, about 2.8 mg, about 2.85 mg, about 2.9 mg, about 2.95 mg, or about 3.0 mg. In a particular embodiment, the invention refers to about 2.5 mg per day of mifepristone or a pharmaceutically acceptable salt, solvate or active metabolite thereof.

The low dose of mifepristone or a pharmaceutically acceptable salt, solvate or active metabolite thereof in combination with the lack of necessity of additional analgesics during the treatment such as NSAIDs (e.g. ibuprofen, naproxen, diclofenac, celecoxib, etc.), paracetamol, metamizole or opioids (e.g. hydrocodone, codeine, etc.) is suitable for a long-term therapy. In a particular embodiment, mifepristone or a pharmaceutically acceptable salt, solvate or active metabolite thereof is administered to a patient for a duration of at least 1, at least 2, at least 3, at least 4, at least 5 or at least 6 months. In another embodiment, mifepristone or a pharmaceutically acceptable salt, solvate or active metabolite thereof is administered to a patient for more than 6 months.

In an embodiment, mifepristone or a pharmaceutically acceptable salt, solvate or active metabolite thereof is administered to a patient that does not have a history of any cancer and/or histological diagnosis of endometrial atypical hyperplasia.

In an additional or alternative embodiment, mifepristone or a pharmaceutically acceptable salt, solvate or active metabolite thereof is administered to a patient that, apart from endometriosis, does not have other clinically significant gynaecological condition causing abnormal uterine bleeding such as adenomyosis or uterine fibroids (also called uterine leiomyomata).

The invention also refers to the use of mifepristone or a pharmaceutically acceptable salt, solvate or active metabolite thereof for the manufacture of a medicament for treatment and/or prevention of endometriosis associated pain at a therapeutically effective amount of between 2 mg and 3 mg per day, wherein said use does not involve additional analgesic agents.

Likewise, the invention refers to a dose regimen for providing analgesia against endometriosis associated pain in a subject by administering mifepristone, or a pharmaceutically acceptable salt, solvate or active metabolite thereof as the one and only analgesic agent at a therapeutically effective amount of between 2 mg and 3 mg per day.

Particular embodiments of the present invention include compounds, compositions, kits, methods, uses and dose regimens as described herein wherein mifepristone or a pharmaceutically acceptable salt, solvate or active metabolite thereof is administered to a patient that meets at least one of the following conditions:
- Premenopausal woman, e.g. between 18 and 45 years of age inclusive; and/or
- Patient with Endometriosis Associated Pelvic Pain (EAPP: i.e. any type of pelvic pain associated with endometriosis: nonmenstrual pelvic pain, dysmenorrhea, dyschezia and dyspareunia) as measured by Visual Analogue Scale (VAS) ≥ 40 mm at least once in the previous 4 weeks; and/or
- Patient with a history of EAPP for at least the past 6 months; and/or
- Endometriosis diagnosed by laparoscopy and/or surgery in the past 10 years or less or diagnosed by MRI or transvaginal ultrasound in the past 5 years; and/or
- Patient with a history of regular menstrual cycles (21-35 days) while not being on any pharmacological treatment that could alter the menstrual cycle (e.g. oral contraceptive pills); and/or
- Patient using double barrier contraception birth control methods (e.g. condom with spermicide) during the entire length of treatment; and/or
- Patient whose sexual partner(s) is (are) vasectomized, at least 6 months prior to treatment; and/or
- Patient has had a bilateral tubal occlusion (including ligation and blockage methods such as Essure^{®}), at least 3 months prior to treatment; and/or
- Patient is not sexually active with men.

Particular embodiments of the present invention include compounds, compositions, kits, methods, uses and dose regimens as described herein wherein mifepristone or a pharmaceutically acceptable salt, solvate or active metabolite thereof is administered to a patient that does not meet at least one of the following conditions:
- History of hypersensitivity or intolerance to the active substance or any of the excipients microcrystalline cellulose, polyvinylpyrrolidone, Croscarmellose sodium, anhydrous colloidal silica or magnesium stearate; and/or
- Subject is pregnant or breast-feeding or is planning a pregnancy during the duration of the treatment or is less than 6 months postpartum, post-abortion, or post-lactation at the time of entry into treatment; and/or
- Previous use of hormonal agents before the treatment: ≤ 24 weeks for GnRH agonists; ≤ 12 weeks for depot progestogens and danazol: ≤ 6 weeks for oral contraceptives; and/or
- Patients with history of any cancer (including breast cancer) and/or histological diagnosis of endometrial atypical hyperplasia (the combination of endometrial thickness <5 mm determined by transvaginal ultrasound and insufficient material for histological evaluation from the endometrial biopsy will be regarded as atrophic endometrium); and/or
- Other clinically significant gynaecological condition causing abnormal uterine bleeding already known or identified on screening such as severe adenomyosis or symptomatic uterine fibroids requiring treatment; and/or
- Subject has current or history of abnormal genital bleeding; and/or
- Presence of ovarian cyst of unknown etiology; and/or
- Cervical smear with pathological findings: class III or higher according to Papanicolaou, class IIp or higher according to the Munich III nomenclature or ASC-US or higher according to the Bethesda system; and/or
- Surgical treatment of endometriosis within 3 months prior to the treatment; and/or
- Presence of chronic pain other than pain due to endometriosis; and/or
- Hepatic insufficiency; and/or
- History of, or complications of, thrombosis, embolism, or serious cardiac, respiratory (severe asthma not adequately controlled by treatment), renal, hematologic, endocrine diseases; and/or
- Uncontrolled or inadequately controlled hypertension with a resting supine systolic or diastolic blood pressure > 180 mmHg or > 95 mmHg, respectively; and/or
- Subject with hereditary porphyria; and/or
- Abnormal laboratory findings considered as clinically significant; and/or
- Positive results for HBs-Ag, anti-HCV and HIV-1/HIV-2-antibodies; and/or
- Use of drugs that could be expected to affect the release of sex hormones (e.g., antipsychotics); hypericum and CYP3A4 inductors (carbamazepine, phenobarbital, phenytoin, primidone, rifampicin) or inhibitors (such as cyclosporine, macrolide antibiotics, nefazodone, azolic antifungal agents and HIV protease inhibitors); and/or
- Intake of any analgesics other than mifepristone or a pharmaceutically acceptable salt, solvate or active metabolite thereof: both opioids and non-steroidal anti-inflammatory drugs as well as paracetamol or metamizole during the treatment period; and/or
- History of drug or alcohol abuse within 6 months prior to treatment.

The skilled person knows that numerical values relating to measurements are subject to measurement errors which place limits on their accuracy. Where terms such as "about" or "approximately" are applied to a particular value (e.g. "about 200 °C" or "approximately 200 °C") or to a range (e.g. "about x to approximately y"), the value or range is interpreted as being as accurate as the method used to measure it. Unless explicitly stated otherwise, the general convention in the scientific and technical literature may be applied so that the last digit of numerical values preferably indicates the precision of measurement. Thus, unless other error margins are given, the maximum margin is preferably ascertained by applying the rounding-off convention to the last decimal place.

For instance, a value of 3.5 preferably has an error margin of 3.45 to 3.54 and a range of 2% to 10% preferably covers a range of 1.5% to 10.4%. Said variations of a specified value are understood by the skilled person and are within the context of the present invention. Further, to provide a more concise description, some of the quantitative expressions given herein are not qualified with the term "about". It is understood that, whether the term "about" is used explicitly or not, every quantity given herein is meant to refer to the actual given value, and it is also meant to refer to the approximation to such given value that would reasonably be inferred based on the ordinary skill in the art, including equivalents and approximations due to the experimental and/or measurement conditions for such given value.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "about 1 % to about 5 %" should be interpreted to include not only the explicitly recited values of about 1 % to about 5 %, but also include individual values and sub-ranges within the indicated range. Thus, included in this numerical range are individual values such as 2, 3. and 4 and sub-ranges such as from 1-3, from 2-4, and from 3-5, etc. This same principle applies to ranges reciting only one numerical value.

All the features described in this specification (including the claims, description and drawings) can be combined in any combination, with the exception of combinations of such mutually exclusive features.

The following examples are merely illustrative of certain embodiments of the invention and cannot be considered as restricting it in any way.

### Examples

### Effect of 2.5 mg mifepristone therapy on endometriosis associated pain

### 1. Clinical trial

A randomized, double-blind, three-arm, parallel-group, multicentre superiority study assessing the efficacy and safety of mifepristone (2.5 mg and 5 mg) vs. placebo for the treatment of endometriosis in reproductive-age women for 24 weeks is ongoing at the date of filing of the first application in respect of the present invention (EudraCT number 2018-002367-26).

The main objective of the trial is to show superiority of at least one dose of mifepristone 2.5 mg and mifepristone 5 mg relative to placebo for the treatment of endometriosis in reproductive-age women at 24 weeks in terms of the reduction of Endometriosis Associated Pelvic Pain (EAPP) as measured by Visual Analog Scale (VAS) and considering intake of rescue medication.

Both mifepristone products were formulated as tablets with the following respective compositions:

| **Tablet composition (Mifepristone 2.5 mg)** | | | |
|---|---|---|---|
| **Component** | **Function** | **Quantity** | **Percentage** |
| Mifepristone | Active ingredient | 2.50 mg | 2.45% |
| Microcrystalline cellulose | Diluent (excipient) | 90.95 mg | 89.17% |
| Polyvinylpyrrolidone (povidone) | Binder (excipient) | 4.00 mg | 3.92% |
| Croscarmellose sodium | Disintegrant (excipient) | 3.20 mg | 3.14% |
| Anhydrous colloidal silica | Glidant (excipient) | 0.30 mg | 0.29% |
| Magnesium stearate | Lubricant (excipient) | 1.05 mg | 1.03% |
| **Total** | | 102.00 mg | 100% |

| **Tablet composition (Mifepristone 5 mg)** | | | |
|---|---|---|---|
| **Component** | **Function** | **Quantity** | **Percentage** |
| Mifepristone | Active ingredient | 5.00 mg | 4.90% |
| Microcrystalline cellulose | Diluent (excipient) | 88.45 mg | 86.72% |
| Polyvinylpyrrolidone (povidone) | Binder (excipient) | 4.00 mg | 3.92% |
| Croscarmellose sodium | Disintegrant (excipient) | 3.20 mg | 3.14% |
| Anhydrous colloidal silica | Glidant (excipient) | 0.30 mg | 0.29% |
| Magnesium stearate | Lubricant (excipient) | 1.05 mg | 1.03% |
| **Total** | | 102.00 mg | 100% |

The planned number of subjects to be included in the whole clinical trial was 490.

The principal inclusion criteria were:
[1] Premenopausal female, between 18 and 45 years of age inclusive, at the time of signing consent
[2] Patients with Endometriosis Associated Pelvic Pain (EAPP: i.e. any type of pelvic pain associated with endometriosis: nonmenstrual pelvic pain, dysmenorrhea, dyschezia and dyspareunia) as measured by Visual Analogue Scale (VAS) ≥ 40 mm at least once in the previous 4 weeks from baseline visit
[3] Patients with a history of EAPP for at least the past 6 months prior to the initial screening visit
[4] Endometriosis diagnosed by laparoscopy and/or surgery in the past 10 years or less or diagnosed by MRI or transvaginal ultrasound in the past 5 years
[5] Patients with a history of regular menstrual cycles (21-35 days) while not being on any pharmacological treatment that could alter the menstrual cycle (e.g. oral contraceptive pills)
[6] Patients who agree to use only ibuprofen 400 mg as rescue medication up to a total daily dose of not more than 2400 mg
[7] Patients willing and able (e.g. mental and physical condition) to participate in all aspects of the study as evidenced by providing signed written informed consent
[8] Patient agrees to use double barrier contraception birth control methods (e.g. condom with spermicide) during the entire length of participation in the study. Patient is not required to use double barrier contraception methods if:
   - Sexual partner(s) is (are) vasectomized, at least 6 months prior to screening
   - Patient has had a bilateral tubal occlusion (including ligation and blockage methods such as Essure^{®}), at least 3 months prior to screening
   - Patient is not sexually active with men; periodic sexual relationship(s) with men requires the use of dual nonhormonal contraception as noted above

The principal exclusion criteria were:
[1] History of hypersensitivity or intolerance to the active substance or any of the excipients of the study medication
[2] Subject is pregnant or breast-feeding or is planning a pregnancy within the duration of the study or is less than 6 months postpartum, post-abortion, or post-lactation at the time of entry into the screening period
[3] Previous use of hormonal agents before the Screening visit: ≤ 24 weeks for GnRH agonists; ≤ 12 weeks for depot progestogens and danazol: ≤ 6 weeks for oral contraceptives
[4] Patients with history of any cancer (including breast cancer) and/or histological diagnosis of endometrial atypical hyperplasia at baseline (note: the combination of endometrial thickness <5 mm determined by transvaginal ultrasound and insufficient material for histological evaluation from the endometrial biopsy will be regarded as atrophic endometrium)
[5] Other clinically significant gynaecological condition causing abnormal uterine bleeding already known or identified on Screening such as severe adenomyosis or symptomatic uterine fibroids requiring treatment
[6] Subject has current or history of abnormal genital bleeding
[7] Presence of ovarian cyst of unknown etiology
[8] Cervical smear with pathological findings: class III or higher according to Papanicolaou, class IIp or higher according to the Munich III nomenclature or ASC-US or higher according to the Bethesda system
[9] Surgical treatment of endometriosis within 3 months prior to the screening visit
[10] Presence of chronic pain other than pain due to endometriosis
[11] Hepatic insufficiency
[12] History of, or complications of, thrombosis, embolism, or serious cardiac, respiratory (severe asthma not adequately controlled by treatment), renal, hematologic, endocrine diseases
[13] Uncontrolled or inadequately controlled hypertension at the time of screening with a resting supine systolic or diastolic blood pressure > 180 mmHg or > 95 mmHg, respectively
[14] Subject with hereditary porphyria
[15] Abnormal laboratory findings considered by the investigator as clinically significant
[16] Positive results for HBs-Ag, anti-HCV and HIV-1/HIV-2-antibodies
[17] Use of drugs that could be expected to affect the release of sex hormones (e.g., antipsychotics); hypericum and CYP3A4 inductors (carbamazepine, phenobarbital, phenytoin, primidone, rifampicin) or inhibitors (such as cyclosporine, macrolide antibiotics, nefazodone, azolic antifungal agents and HIV protease inhibitors)
[18] Intake of any analgesics other than ibuprofen: both opioids and non-steroidal anti-inflammatory drugs as well as paracetamol or metamizole during the screening period
[19] History of drug or alcohol abuse within 6 months prior to screening
[20] Participation in another trial within 3 months from the screening visit date
[21] Previous enrolment in this study
[22] Any condition that, in the judgment of the investigator, may interfere with adherence to study procedures or study assessments
[23] Legal incapacity and/or other circumstances rendering the patient unable to understand the nature, scope and possible consequences of the study
[24] Unreliability or lack of cooperation during the screening and baseline period (Note that this includes proven ability to use the e-diaries via the internet).

### 2. Results

Surprisingly, it has been found that those patients receiving a mifepristone daily dose of 2.5 mg (Treatment Group T1) alone (with no analgesic rescue medication) experienced a significant reduction in the intensity of pain associated with endometriosis, more pronounced than the 5 mg group (Treatment Group T2).

Table 1 and Figure 1 show the evolution of endometriosis associated pain (EAP) in both groups of patients as measured by the visual analogue scale (VAS) before and at 1, 2, 3 and 4 months of treatment.

| | EAP (VAS) | |
|---|---|---|
| Months | T1 (2.5 mg) | T2 (5 mg) |
| 0 | 32.81 | 32.81 |
| 1 | 29.19 | 29.30 |
| 2 | 24.61 | 26.11 |
| 3 | 23.63 | 24.58 |
| 4 | 22.31 | 25.01 |

It was also found that the 2.5 mg mifepristone daily dose was effective to reduce the pain associated with extra-pelvic endometriosis. For instance, patients with endometrial lesions in cesarean sections (C-sections) or ovarian endometriomas taking a 2.5 mg mifepristone tablet every day as monotherapy reported appropriate pain relief.

### List of references:

CN106317152A
Carbonell J L et al. Mifepristone 2.5, 5, 10 mg versus placebo in the treatment of endometriosis. Journal of Reproductive Health and Medicine 2016; 2(1): 17-25
ClinicalTrials.gov Identifier NCT02271958
EudraCT number 2018-002367-26
Kettel L M, et al. Endocrine responses to long-term administration of the anti progesterone RU486 in patients with pelvic endometriosis. Fertil Steril 1991; 56:402-7.
Kettel L M, et al. Treatment of endometriosis with the antiprogesterone mifepristone (RU486). Fertil Steril 1996; 65:23-8.
Kettel L M, et al. Preliminary report on the treatment of endometriosis with low-dose mifepristone (RU 486). Am J Obstet Gynecol 1998; 178:1151-6
Shi YE, Ye ZH, He CH, Zhang GQ, Xu JQ, Van Look PF, Fotherby K. Pharmacokinetic study of RU 486 and its metabolites after oral administration of single doses to pregnant and non-pregnant women. Contraception 1993; 48(2): 133-49
Xu, Juan, Li, Peng, Wang, Hui P. and Ning, Li F.. "Crystal structure of 11-(4-(dimethylamino)phenyl)-17-hydroxy-13-methyl-17-(prop-1-yn-1-yl)-1,2,6,7,8,11,12,13,14,15,16,17-dodecahydro-3H-cyclopenta[a]phenanthren-3-one - acetonitril (1/2), C33H41N3O2" Zeitschrift fur Kristallographie - New Crystal Structures, vol. 232, no. 4, 2017, pp. 573-575

## Claims

1. Mifepristone or a pharmaceutically acceptable salt, solvate or active metabolite thereof for use in the treatment and/or prevention of endometriosis associated pain, wherein said mifepristone or a pharmaceutically acceptable salt, solvate or active metabolite thereof is used as the sole analgesic agent at a daily dose of between 2 mg and 3 mg.

2. Mifepristone or a pharmaceutically acceptable salt, solvate or active metabolite thereof for use according to claim 1, wherein the daily dose is about 2.5 mg.

3. A pharmaceutical composition for use in the treatment and/or prevention of endometriosis associated pain, said composition comprising mifepristone, or a pharmaceutically acceptable salt, solvate or active metabolite thereof and a pharmaceutically acceptable excipient, and wherein said mifepristone or a pharmaceutically acceptable salt, solvate or active metabolite thereof is used as the sole analgesic agent at a daily dose of between 2 mg and 3 mg.

4. The pharmaceutical composition for use according to claim 3, wherein said composition is for oral administration.

5. The pharmaceutical composition for use according to any one of claims 3 or 4, wherein said composition is a tablet.

6. The pharmaceutical composition for use according to any one of claims 3 to 5, wherein the daily dose is about 2.5 mg.

7. A pharmaceutical composition in the form of a tablet for oral administration comprising:
Mifepristone or a pharmaceutically acceptable salt, solvate or active metabolite thereof 2 - 3 mg
Microcrystalline cellulose 77 - 95 mg
Polyvinylpyrrolidone 2 - 6 mg
Croscarmellose sodium 1 - 5 mg
Anhydrous colloidal silica 0 - 1 mg
Magnesium stearate 0 - 2 mg.

8. A pharmaceutical composition in the form of a tablet for oral administration comprising:
Mifepristone or a pharmaceutically acceptable salt, solvate or active metabolite thereof, between more than 2 % w/w and less than 3 % w/w
Microcrystalline cellulose 76 - 94 % w/w
Polyvinylpyrrolidone 2 - 6 % w/w
Croscarmellose sodium 1 - 5 % w/w
Anhydrous colloidal silica 0 -1 % w/w
Magnesium stearate 0 - 2 % w/w.

9. The pharmaceutical composition according to any one of claims 7 or 8 for use in the treatment and/or prevention of endometriosis associated pain.

10. The pharmaceutical composition according to any one of claims 7 or 8 or the pharmaceutical composition for use according to claim 9, wherein the composition is administered to provide a daily dose of between 2 mg and 3 mg of mifepristone or a pharmaceutically acceptable salt, solvate or active metabolite thereof.

11. The pharmaceutical composition according to any one of claims 7 or 8 or the pharmaceutical composition for use according to claim 9, wherein the composition is administered to provide a daily dose of about 2.5 mg of mifepristone or a pharmaceutically acceptable salt, solvate or active metabolite thereof.

12. A kit comprising mifepristone or a pharmaceutically acceptable salt, solvate or active metabolite thereof, preferably provided as a pharmaceutical composition, and instructions for use in the treatment and/or prevention of endometriosis associated pain at a daily dose of between 2 mg and 3 mg of mifepristone or a pharmaceutically acceptable salt, solvate or active metabolite thereof.

13. A kit according to claim 12, wherein the daily dose is about 2.5 mg.
